# EUROPEAN PATENT APPLICATION

(11) **EP 4 636 056 A1**
(43) Date of publication of application: **22.10.2025**
(21) Application number: 23903054.7
(22) Date of filing: 11.09.2023
(51) Int. Cl.: C09K 23/56, A23L 29/00, A23L 33/15, A61K 8/73, C01B 32/168, C01B 32/194, C08B 37/14

(54) **DISPERSING AGENT FOR POORLY SOLUBLE OR INSOLUBLE SUBSTANCE, AND DISPERSION LIQUID**

(30) Priority: 13.12.2022 JP 2022198866
(71) Applicant: Seiwa Electric MFG. Co., Ltd., Joyo-shi, Kyoto 610-0192 (JP)
(72) Inventor: SUZUKI Shiho, Joyo-shi Kyoto 610-0192 (JP); MATSUNO Kenji, Joyo-shi Kyoto 610-0192 (JP); TAKAGI Kai, Joyo-shi Kyoto 610-0192 (JP)
(74) Representative: Herrero & Asociados, S.L.
(86) International application number: PCT/JP2023/032949
(87) International publication number: WO 2024/127752

(57) **Abstract**

Provided is a dispersing agent with a good dispersing ability and environmental friendliness by using carboxymethylated xylan obtained through the carboxymethylation of xylan, for the dispersion of poorly soluble or insoluble substances, and a dispersion liquid. The present invention is characterized by using carboxymethylated xylan obtained by carboxymethylating xylan. In this case, the xylan may be glucuronoxylan. It is preferable that the carboxymethylated xylan has a degree of substitution of carboxymethylated xylan (degree of CM substitution) 0.1 or more and 0.4 or less. If the carboxymethylated xylan is obtained by carboxymethylating glucuronoxylan and has a degree of CM substitution of 0.1 or more and 0.4 or less, it can be used as a dispersing agent with an excellent dispersing ability. The poorly soluble or insoluble substance may be carbon nanotubes, graphene, β-carotene, or phthalocyanine.

## Description

### Technical Field

The present invention mainly relates to a dispersing agent and a dispersion liquid for dispersing a poorly soluble or insoluble substance such as carbon nanotubes in water.

### Background Art

Carbon nanotubes (hereafter, referred to as "CNTs") are cylindrical (tubelike) substances made only of carbon and having a nanometer-sized diameter, and have a structure in which a planar sheet on which benzene rings are all arranged next to each other is rolled into a cylindrical shape, the benzene rings being each constituted by carbon atoms arranged in a hexagonal shape. A carbon nanotube constituted by one such cylindrical layer is referred to as a single-walled carbon nanotube (hereafter, referred to as a "SWCNT"), and a carbon nanotube constituted by a plurality of such cylindrical layers having different diameters and arranged one inside another is referred to as a multi-walled carbon nanotube (hereafter, referred to as an "MWCNT").

Carbon nanotubes have high electrical conductivity and great mechanical strength, and research is being conducted on their applications utilizing such properties to conductive paints, conductive resins, electromagnetic shielding sheets, heater components, and the like. The dispersibility of CNTs is important for these applications. The reason for this is that in the solid state, CNTs form a bundle structure due to strong π-π interactions and van der Waals forces, making them difficult to disperse in many solvents. Therefore, in order to make CNTs dispersible in solvents and enable various applications, an excellent dispersing agent is needed to assist in this process.

For example, it has been disclosed that a sheet having excellent electromagnetic wave suppression and heat generation properties is produced by coating a sheet substrate with an aqueous dispersion liquid in which CNTs have been added to and dispersed in an aqueous solution containing a dispersing agent constituted by a specific anionic surfactant and a specific polysaccharide. The aqueous dispersion liquid contains a dispersing agent constituted by: one or more types of anionic surfactants selected from an A group consisting of methylnaphthalene sulfonate formalin condensate salt, naphthalene sulfonate formalin condensate salt, and alkylene maleate copolymer salt; and one or more types of polysaccharides selected from a B group consisting of water-soluble xylan, xanthan gums, guar gums, gellan gums, and carboxymethylcellulose (see Patent Document 1, for example).

It has also been disclosed that the use of water-soluble xylan is found to improve the affinity of a solvent to the surface of a poorly soluble or insoluble substance, based on which the affinity of a solvent to the surface of a poorly water-soluble or water-insoluble substance is improved to obtain a solution of that substance (see Patent Document 2, for example).

Researchers including the inventors of the present invention found that methylglucuronoxylan, which is a hemicellulose, has the function of dispersing hydrophobic substances in water, and have studied the dispersion mechanism of CNTs and their applications to silicone rubber for shielding electromagnetic waves (see Non-Patent Document 1, for example).

### Citation List

### Patent Documents

Patent Document 1: JP 2013-082610A
Patent Document 2: JP 2007-215542A

### Non-Patent Document

Non-Patent Document 1: Journal of Applied Glycoscience, Volume 12, Issue 1, 27-32 (2022)

### Summary of Invention

### Technical Problem

The invention described in Patent Document 1 discloses a carbon nanotube aqueous dispersion liquid using a dispersing agent constituted by one or more types of specific anionic surfactants from the A group and one or more types of specific polysaccharides from the B group. The viscosity of CNT dispersion liquids increases when the CNT concentration is increased in the case of using a dispersing agent constituted only by a polysaccharide such as carboxymethylcellulose, for example, and thus this invention adds an anionic surfactant to the dispersing agent in order to suppress such an increase in the viscosity, thereby making it possible to use the dispersion liquids for coating. However, the addition of a surfactant may cause air bubbles, which requires attention in good coating film formation work.

The invention described in Patent Document 2 states that water-soluble xylan makes it possible to disperse a poorly soluble or insoluble substance. It is often required to increase the degree of dispersion of CNTs in order to apply CNT dispersion liquids to electronic materials. However, it is difficult to further increase the degree of dispersion using only water-soluble xylan.

The invention described in Non-Patent Document 1 shows an example in which a dispersion liquid of SWCNTs dispersed using a dispersing agent constituted by water-soluble xylan, which is a natural product, is prepared and used as a paint for applications to electronic materials . However, it is difficult to further increase the degree of dispersion by merely using a natural product.

Environmental issues are currently a very important topic, and even materials with very excellent properties cannot be used if they have a large environmental impact. Water is the most suitable solvent when environmental impact and biocompatibility are taken into consideration. Dispersing agents used to dissolve poorly water-soluble substances are also required to be environmentally friendly or biocompatible materials. Therefore, it is preferable that the dispersing agents used to disperse poorly soluble or insoluble substances are also natural products or biodegradable compounds.

It is an object of the present invention to provide a dispersing agent with a good dispersing ability and environmental friendliness by using carboxymethylated xylan obtained through the carboxymethylation of xylan, for the dispersion of poorly soluble or insoluble substances, and a dispersion liquid.

### Solution to Problem

In order to solve the above-described conventional problems, a dispersing agent for a poorly soluble or insoluble substance of the present invention is characterized by using carboxymethylated xylan obtained through the carboxymethylation of xylan.

In this case, the xylan may be glucuronoxylan. It is preferable that the carboxymethylated xylan has a degree of substitution of carboxymethylated xylan (hereafter, referred to as "degree of CM substitution") of 0.1 or more and 0.4 or less.

If the carboxymethylated xylan is obtained through the carboxymethylation of xylan, in particular glucuronoxylan and has degree of CM substitution of 0.1 or more and 0.4 or less, it can be used as a dispersing agent with an excellent dispersing ability.

In this case, the poorly soluble or insoluble substance may be carbon nanotubes, graphene, β-carotene, or phthalocyanines. Despite the fact that these substances have various high-performance properties, their applications are limited because they cannot be dispersed in water or other solvents. The dispersing agent of the present invention enables applications of these substances in a wide range of fields.

Furthermore, a carbon nanotube dispersion liquid of the present invention contains the above-described dispersing agent, a poorly soluble or insoluble substance constituted by carbon nanotubes, and water. Since such a dispersion liquid can allow carbon nanotubes to be dispersed therein and keep a stable dispersing ability, it can be applied to a wide range of fields, including the field of electronic materials such as conductive materials and electromagnetic shielding sheets, and the field of batteries.

Moreover, a graphene dispersion liquid of the present invention contains the above-described dispersing agent, a poorly soluble or insoluble substance constituted by graphene, and water. Such a graphene dispersion liquid can be applied to a substrate surface to produce conductive coatings, electromagnetic shielding materials, field emission materials, and the like.

Moreover, a β-carotene dispersion liquid of the present invention contains the above-described dispersing agent, a poorly soluble or insoluble substance constituted by β-carotene, and water. Since carboxymethylated xylan obtained through the carboxymethylation of glucuronoxylan is also a material whose impact on human safety and environment is low, the β-carotene dispersion liquid using this dispersing agent can be applied as cosmetic materials or food additives.

Moreover, a phthalocyanine dispersion liquid of the present invention contains the above-described dispersing agent, a poorly soluble or insoluble substance constituted by phthalocyanine, and water. Phthalocyanine is often used as pigments, and the use of this dispersion liquid with an enhanced dispersing ability facilitates their applications to various colorants.

### Advantageous Effects of Invention

The dispersing agent for a poorly soluble or insoluble substance and the dispersion liquid of the present invention can easily disperse poorly soluble or insoluble substances such as CNTs, and, in particular a dispersion liquid in which CNTs are dispersed has large effects in the field of electronic components.

### Brief Description of Drawings

FIG. 1 shows results of a relationship between the degree of CM substitution of carboxymethylated xylan and the degree of dispersion of MWCNTs, in Example 1 according to this embodiment.
FIG. 2 shows research results of the degree of dispersion and the viscosity with respect to the degree of CM substitution when the amount of MWCNTs added was small (0.1 wt%), in Example 1 according to this embodiment.
FIG. 3 shows research results of the degree of dispersion and the viscosity with respect to the degree of CM substitution when the amount of MWCNTs added was large (1 wt%), in Example 1 according to this embodiment.
FIG. 4 is a diagram showing results of dispersion of SWCNTs in comparison with results of dispersion of MWCNTs, in Example 1 according to this embodiment.
FIG. 5 is a diagram showing results of dispersion of graphene in comparison with results of dispersion of MWCNTs, in Example 1 according to this embodiment.
FIG. 6 is a diagram showing results of dispersion of copper phthalocyanine, obtained by measuring and evaluating the absorption spectra using a UV-visible spectrophotometer, in Example 1 according to this embodiment.
FIG. 7 is a diagram showing results of dispersion of copper phthalocyanine, obtained by measuring and evaluating the absorption spectra using a UV-visible spectrophotometer, in Example 1 according to this embodiment.

### Description of Embodiments

### Embodiment

Hereafter, a dispersing agent for a poorly soluble or insoluble substance according to an embodiment of the present invention will be described. The dispersing agent for a poorly soluble or insoluble substance of the present invention is characterized by using carboxymethylated xylan obtained through the carboxymethylation of xylan. The inventors of the present invention have already found that glucuronoxylan has good properties as a dispersing agent for poorly soluble or insoluble substances, and they have made various efforts to further improve the dispersing ability and the dispersion stability. As a result, they found that carboxymethylated xylan obtained through the carboxymethylation of glucuronoxylan as xylan has even better properties as a dispersing agent. Furthermore, they found that it is preferable that the degree of CM substitution is 0.1 or more and 0.4 or less in the carboxymethylation, and thus completed the present invention. Note that the present invention is not limited to glucuronoxylan as xylan, and the same effect can be obtained with arabinoglucuronoxylan, glucuronoarabinoxylan, arabinoxylan, or the like, described later.

In the above-described invention, the poorly soluble or insoluble substance may be carbon nanotubes, graphene, β-carotene, or phthalocyanine. These substances are known to be poorly soluble or insoluble in water, and good dispersing agents are required. The dispersing agent of the present invention allows dispersion with a large degree of dispersion in water. Furthermore, the carboxymethylated xylan is obtained through the carboxymethylation of glucuronoxylan and is an environmentally friendly material, and when used as a dispersing agent, it can not only improve the dispersibility of poorly soluble substances such as carbon nanotubes, graphene, β-carotene, and phthalocyanine, but also produce environmentally friendly dispersion liquids.

Furthermore, the carbon nanotube dispersion liquid of the present invention contains the above-described dispersing agent, a poorly soluble or insoluble substance constituted by carbon nanotubes, and water. Such a carbon nanotube dispersion liquid can be used for purposes such as conductive coatings, electromagnetic shielding materials, field emission materials, and anode materials for batteries.

Furthermore, the graphene dispersion liquid of the present invention contains the above-described dispersing agent, a poorly soluble or insoluble substance constituted by graphene, and water. Such a graphene dispersion liquid can be applied to a substrate surface to produce conductive coatings, electromagnetic shielding materials, field emission materials, and the like.

Moreover, the β-carotene dispersion liquid of the present invention contains the above-described dispersing agent, a poorly soluble or insoluble substance constituted by β-carotene, and water. Since carboxymethylated xylan obtained through the carboxymethylation of glucuronoxylan is also a material whose impact on human safety and environment is low, the β-carotene dispersion liquid using this dispersing agent can be applied as cosmetic materials or food additives.

Moreover, the phthalocyanine dispersion liquid of the present invention contains the above-described dispersing agent, a poorly soluble or insoluble substance constituted by phthalocyanine, and water. Phthalocyanine is often used as pigments, and the use of this dispersion liquid with an enhanced dispersing ability facilitates their applications to various colorants.

Phthalocyanine is chemically a cyclic compound with four isoindoles, and is chemically called phthalocyanine when there is no metal in the center, but there are many with a metal in the center, of which copper phthalocyanine is a typical example. In the present invention, those including copper phthalocyanine, cobalt phthalocyanine, zinc phthalocyanine, magnesium(II) phthalocyanine, tin(II) phthalocyanine, metal-free phthalocyanine, low-chlorinated phthalocyanine, and the like are referred to as phthalocyanines.

Note that the degree of CM substitution of carboxymethylated xylan refers to the number of hydroxyl groups substituted with carboxymethyl groups, out of the two hydroxyl groups present per xylose unit.

Xylan refers to molecules containing two or more xylose residues linked by β-1,4 bonds. Molecules constituted only by xylose residues (i.e., pure xylose polymers) as well as xylose polymers to which 4-O-methylglucuronic acid residues and acetyl groups are attached are generally referred to as glucuronoxylan. It is known that glucuronoxylan is the main component of hemicellulose contained in hardwood wood. Glucuronoxylan contained in hardwoods is often constituted by xylose residues, 4-O-methylglucuronic acid residues, and acetyl groups in a ratio of 10:1:6. Some xylose polymers have arabinose residues and 4-O-methylglucuronic acid attached thereto. These are generally referred to as arabinoglucuronoxylan or glucuronoarabinoxylan. Xylose polymers to which arabinose residues are attached are generally referred to as arabinoxylan. In the present invention, these are collectively referred to as xylan.

### Example 1

In this example, results of dispersing MWCNTs using carboxymethylated xylan obtained through the carboxymethylation of glucuronoxylan as xylan will be described. First, the procedure for the substitution of carboxymethylation of glucuronoxylan will be described.

100 g of ethanol, 10 g of 30% sodium hydroxide solution, and 3 g of sodium monochloroacetate were added to 10 g of glucuronoxylan, and the mixture was stirred at 45°C. After 15, 30, and 60 minutes, respectively, a portion of the reaction solution was collected and ethanol was added to obtain a precipitate. This precipitate was washed with 90% ethanol and dried at 70°C to produce carboxymethylated xylan with different degrees of substitution of carboxymethylated xylan.

The degree of substitution of carboxymethylated xylan (which may be hereafter referred to as "degree of CM substitution") of the produced carboxymethylated xylan was calculated as follows. 200 mg of carboxymethylated xylan with different degrees of CM substitution were each suspended in 2 mL of 80% ethanol. Furthermore, 2 mL of hydrochloric acid (HCl) was added, and the mixture was stirred for 1 hour and then applied in a centrifuge at 6000 G for 10 minutes to obtain precipitates. These precipitates were washed twice with 80% ethanol, 20 mL of purified water was added thereto, and the mixture was stirred. Furthermore, 25 mL of 0.1 M sodium hydroxide (NaOH) solution was added, and the mixture was heated for 15 minutes. These solutions were titrated with 0.1 M hydrochloric acid (HCl) solution, and the degree of etherification was calculated from the amount of hydrochloric acid added. The degree of CM substitution was then calculated from the difference from the degree of etherification of the sample before carboxymethylation.

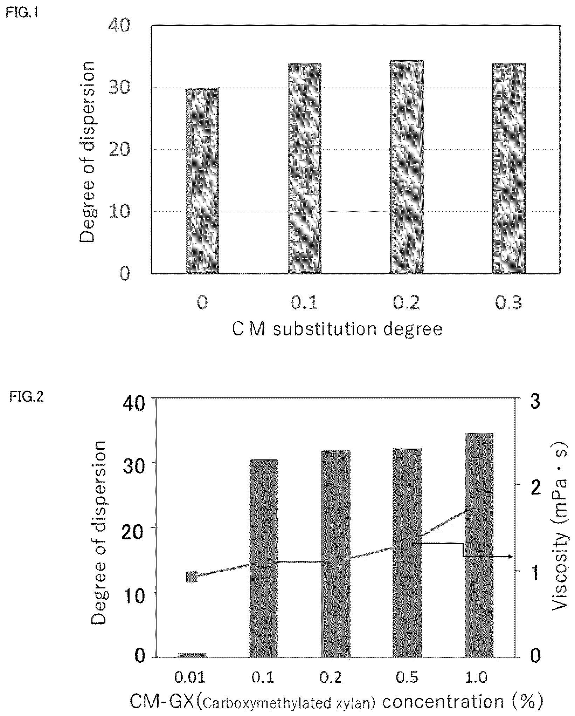

FIG. 1 shows results of a relationship between the degree of CM subustitution of carboxymethylated xylan and the degree of dispersion of MWCNTs. 50 mg of MWCNTs were added to each of 50 mL of aqueous solutions of carboxymethylated xylan with different degrees of CM substitution as described above and dispersed using an ultrasonic homogenizer (600 W). Then, the mixtures were each separated using a centrifuge (CT18R manufactured by Eppendorf Himac Technologies Co., Ltd.) at 10,000 G for 1 hour, and the supernatant was used as the sample. The supernatant was measured for an absorbance at a wavelength of 500 nm using a UV-visible spectrophotometer (UV-1900 manufactured by Shimadzu Corporation), and the value calculated based on the obtained the absorbance was used as the degree of dispersion.

As can be seen from the figure, an improvement in the degree of dispersion was observed when the degree of CM substitution was 0.1 or more, compared with when the degree of CM substitution was 0, that is, when glucuronoxylan was used. Furthermore, although not shown in the figure, it was found that as a result of producing carboxymethylated xylan with large degrees of CM substitution and obtaining the degrees of dispersion of MWCNTs, the degree of dispersion decreases if the degree of CM substitution is more than 0.4. As a result, it was found that the degree of CM substitution is preferably 0.1 or more and 0.4 or less. In particular, the degree of CM substitution is more preferably 0.1 or more and 0.2 or less.

The carboxymethylated xylan can be used without any limitation if its molecular weight is in the range of 1,000 to 1,000,000. In particular, the molecular weight is preferably 1,000 to 30,000, and more preferably 5,000 to 25,000.

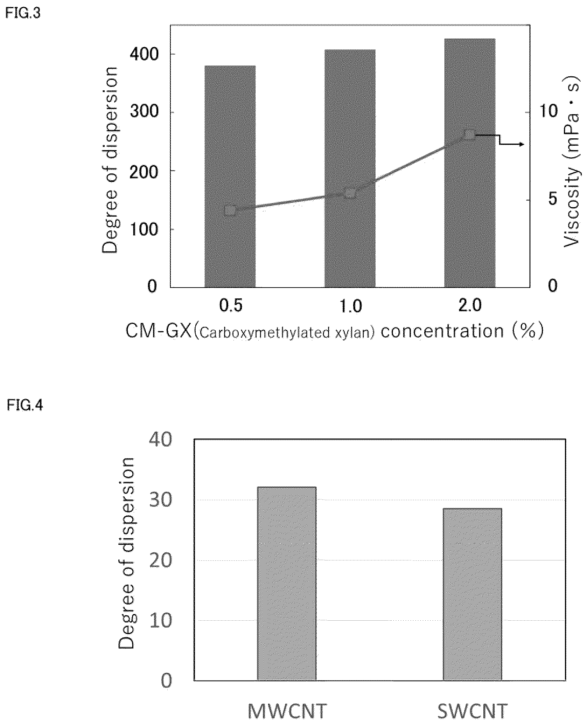

FIGS. 2 and 3 show results of the degree of dispersion and the viscosity when using carboxymethylated xylan with a degree of CM substitution of 0.2, at different concentrations of carboxymethylated xylan in water and different amounts of MWCNTs added.

FIG. 2 shows research results of the degree of dispersion and the viscosity with respect to the degree of CM substitution when the amount of MWCNTs added was small (0.1 wt%). Specifically, aqueous solutions with concentrations of carboxymethylated xylan in water of 0.01 wt%, 0.1 wt%, 0.2 wt%, 0.5 wt%, and 1.0 wt% were prepared using carboxymethylated xylan with degree of CM substitution of 0.2, MWCNTs were added in an amount of 0.1 wt% to produce dispersion liquids, and their degrees of dispersion and viscosities were obtained.

It was seen that MWCNTs were hardly dispersed when the concentration of carboxymethylated xylan in water was 0.01 wt%, but the degree of dispersion rapidly increased when the concentration was increased to 0.1 wt% or more. Furthermore, the degree of dispersion gradually increased when the concentration of carboxymethylated xylan was in the range of 0.1 wt% to 1.0 wt%. Meanwhile, the viscosity was 0.9 mPa·s when the concentration of carboxymethylated xylan was 0.01 wt%, was approximately 1.1 mPa·s when the concentration was 0.1 wt%, and was approximately 1.7 mPa·s even when the concentration was 1.0 wt%. When the amount of MWCNTs added is small, if the concentration of carboxymethylated xylan in water is approximately 0.1 wt%, a sufficient degree of dispersion can be obtained and the use of such carboxymethylated xylan in the production of conductive materials and the like can prevent the viscosity increase and gelation of the dispersion liquid. It also makes the dispersion liquid easier to handle.

FIG. 3 shows research results of the degree of dispersion and the viscosity with respect to the degree of CM substitution when the amount of MWCNTs added was large (1 wt%). Specifically, aqueous solutions with concentrations of carboxymethylated xylan in water of 0.5 wt%, 1.0 wt%, and 2.0 wt% were prepared using carboxymethylated xylan with degree of CM substitution of 0.2, MWCNTs were added in an amount of 1 wt%, which was 10 times the amount in FIG. 2, to produce dispersion liquids, and their degrees of dispersion and viscosities were obtained.

The concentrations of carboxymethylated xylan of 0.5 wt% and 1.0 wt% in FIG. 2 are the same concentrations of carboxymethylated xylan in water respectively as the concentrations of carboxymethylated xylan of 0.5 wt% and 1.0 wt% in FIG. 3. When the amount of MWCNTs added was 1 wt%, the degree of dispersion increased in accordance with an increase in the amount added, and the degree of dispersion was much larger than when the amount added was 0.1 wt%. Specifically, the degree of dispersion was 370 when the concentration of carboxymethylated xylan was 0.5 wt%, was 405 when the concentration was 1.0 wt%, and was 430 when the concentration was 2.0 wt%. Furthermore, it was found that the increased degree of dispersion of MWCNTs also resulted in a larger viscosity of the dispersion liquid even when the concentration of carboxymethylated xylan was the same as that in FIG. 2. Specifically, the viscosity was 4 mPa·s when the concentration of carboxymethylated xylan was 0.5 wt%, and was 5.5 mPa·s when the concentration was 1.0 wt%.

As described above, it was found that if MWCNTs are added in large amounts, compared with when the amount of MWCNTs added is small, the degree of dispersion can be significantly increased and the viscosity can be kept low, and thus the resulting dispersion liquid is effective for applications to conductive materials and the like. In particular, the viscosity of the dispersion liquid is preferably 6 mPa·s or less because it makes the dispersion liquid easier to handle due to its high fluidity and ability to improve work efficiency during application.

### Example 2

In this example, the case in which SWCNTs are used as the CNTs will be described. FIG. 4 shows results of dispersion of SWCNTs in comparison with results of dispersion of MWCNTs. A dispersion liquid containing a dispersing agent using carboxymethylated xylan with degree of CM substitution of 0.2 and a concentration in water of 0.2 wt% was used. 50 mg of SWCNTs were added to 50 mL of this dispersion liquid and dispersed using an ultrasonic homogenizer (600 W). This procedure is the same as for MWCNTs, and thus a description thereof has been omitted. FIG. 4 shows the degree of dispersion of MWCNTs as well for comparison.

As can be seen from FIG. 4, the degree of dispersion of SWCNTs was 28.5, which is smaller than that of MWCNTs but is sufficient for practical use. Note that the SWCNTs particularly preferably have an aspect ratio in the range of 5000 to 100,000.

### Example 3

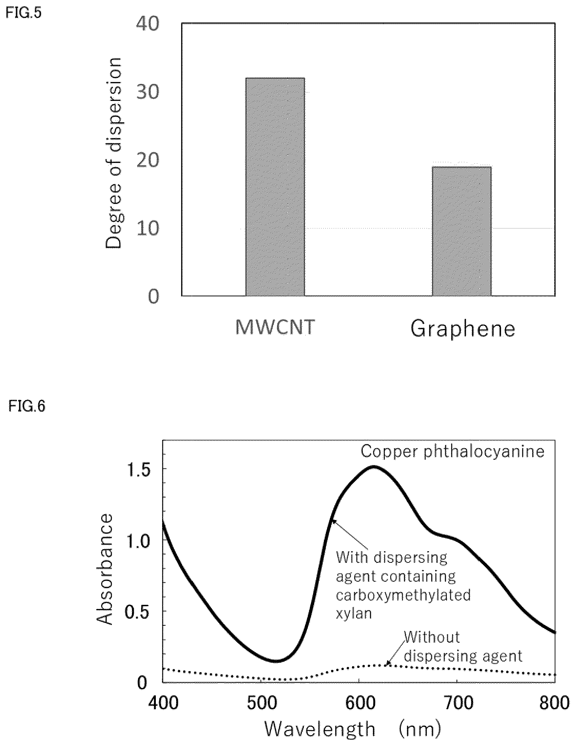

In this example, the case in which graphene is used will be described. FIG. 5 shows results of dispersion of graphene in comparison with results of dispersion of MWCNTs. A dispersion liquid containing a dispersing agent using carboxymethylated xylan with a degree of CM substitution of 0.2 and a concentration in water of 0.2 wt% was used. 50 mg of graphene was added to 50 mL of this dispersion liquid and dispersed using an ultrasonic homogenizer (600 W). This procedure is the same as for MWCNTs, and thus a description thereof has been omitted. FIG. 5 shows the degree of dispersion of MWCNTs as well for comparison. As can be seen from FIG. 5, the degree of dispersion of graphene was 18.9, which is smaller than that of MWCNTs, but is sufficient for practical use.

### Example 4

In this example, the case in which copper(II) phthalocyanine (β-form) is used as the phthalocyanine will be described. In this example, a dispersing agent using carboxymethylated xylan with a degree of CM substitution of 0.2 was produced. This dispersing agent was added to water to 0.2 wt% to produce a 0.2 wt% aqueous solution. 1 mg of copper(II) phthalocyanine (β-form) was added to this aqueous solution and dispersed using an ultrasonic homogenizer (600 W). In this example, the absorption spectra were measured and evaluated using a UV-visible spectrophotometer (UV-1900 manufactured by Shimadzu Corporation) instead of obtaining the degree of dispersion. FIG. 6 shows the results.

As can be seen from FIG. 6, when copper(II) phthalocyanine (β-form) was directly added to water without a dispersing agent, little absorption occurred in the UV-visible spectrophotometer measurement because copper(II) phthalocyanine (β-form) is not soluble in water. However, it was seen that in the case of the dispersion liquid with the dispersing agent added, the absorbance at a wavelength of 500 nm was approximately 0.17, indicating that the dispersion occurred although the degree of dispersion was smaller than that of CNTs.

### Example 5

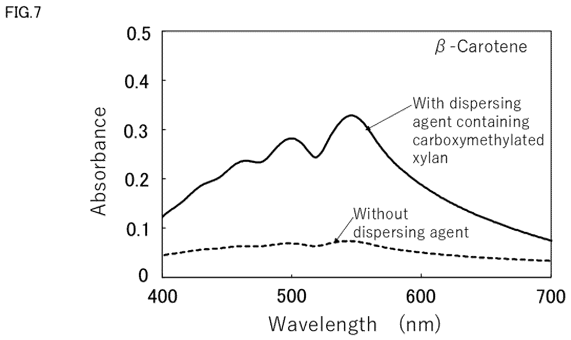

In this example, the case in which β-carotene is used will be described. In this example, a dispersing agent using carboxymethylated xylan with degree of CM substitution of 0.2 was produced. This dispersing agent was added to water to 0.2 wt% to produce a 0.2 wt% aqueous solution. 10 mg of β-carotene was added to this aqueous solution and dispersed using an ultrasonic homogenizer (600 W). In this example, the absorption spectra were measured and evaluated using a UV-visible spectrophotometer (UV-1900 manufactured by Shimadzu Corporation) instead of obtaining the degree of dispersion. FIG. 7 shows the results.

As can be seen from FIG. 7, when β-carotene was directly added to water without a dispersing agent, little absorption occurred in the UV-visible spectrophotometer measurement because β-carotene is not soluble in water. However, it was seen that in the case of the dispersion liquid with the dispersing agent added, the absorbance at a wavelength of 500 nm was 0.275, indicating that the degree of dispersion was good.

Note that "xylan" as used in the present invention includes not only glucuronoxylan, but also arabinoglucuronoxylan, glucuronoarabinoxylan, arabinoxylan, and the like. In the present embodiment and examples, the cases were described in which carboxymethylated xylan obtained by carboxymethylating glucuronoxylan was used as the dispersing agent, but the present invention is not limited to these. Those obtained by carboxymethylating xylan other than glucuronoxylan, such as arabinoxylan and arabinoglucuronoxylan, can also be used as a good dispersing agent for poorly soluble or insoluble substances.

### Industrial Applicability

The dispersing agent for a poorly soluble or insoluble substance and the dispersion liquid of the present invention are useful in the field of electronic components such as electromagnetic shielding sheets utilizing conductivity thereof when CNTs are used, in the fields of food and cosmetics when β-carotene is used, and in the field of paints when phthalocyanine is used.

## Claims

1. A dispersing agent for a poorly soluble or insoluble substance, using carboxymethylated xylan obtained through the carboxymethylation of xylan.

2. The dispersing agent for a poorly soluble or insoluble substance according to claim 1, wherein the xylan is glucuronoxylan.

3. The dispersing agent according to claim 1, wherein the carboxymethylated xylan has degree of CM substitution 0.1 or more and 0.4 or less.

4. The dispersing agent according to any one of claims 1 to 3, wherein the poorly soluble or insoluble substance is carbon nanotubes, graphene, β-carotene, or phthalocyanine.

5. A carbon nanotube dispersion liquid comprising the dispersing agent according to any one of claims 1 to 3, a poorly soluble or insoluble substance constituted by carbon nanotubes, and water.

6. A graphene dispersion liquid comprising the dispersing agent according to any one of claims 1 to 3, a poorly soluble or insoluble substance constituted by graphene, and water.

7. A β-carotene dispersion liquid comprising the dispersing agent according to any one of claims 1 to 3, a poorly soluble or insoluble substance constituted by β-carotene, and water.

8. A phthalocyanine dispersion liquid comprising the dispersing agent according to any one of claims 1 to 3, a poorly soluble or insoluble substance constituted by phthalocyanine, and water.
